# EUROPEAN PATENT SPECIFICATION

(11) **EP 0 862 581 B1**
(45) Date of publication and mention of the grant of the patent: **04.06.2003**
(21) Application number: 96936680.6
(22) Date of filing: 17.10.1996
(51) Int. Cl.: C07K 14/505

(54) **HEPARIN CHROMATOGRAPHY OF ERYTHROPOIETINS**
HEPARINCHROMATOGRAPHIE VON ERYTHROPOIETINEN
CHROMATOGRAPHIE D'ERYTHROPOIETINES PAR HEPARINE

(30) Priority: 08.11.1995 US 555239
(43) Date of publication of application: 09.09.1998
(73) Proprietor: Aventis Pharmaceuticals Inc., Bridgewater, NJ 08807-0800 (US)
(72) Inventor: BOROWSKI, Marianne, Winthrop, MA 02152 (US)
(74) Representative: VOSSIUS & PARTNER
(86) International application number: US9616711
(87) International publication number: WO97017366

(56) References cited:
- GB-A- 886 712

## Description

The present invention is directed to a simple and efficient process for the recovery of erythropoietins from biological and culture fluids using Heparin Chromatography. The present invention can be used to obtain research and commercial quantities of erythropoietins.

Heparin is a highly sulfated glycosaminoglycan. Specifically, Heparin is a mucopolysaccharide consisting of a repeating dimer of L-dipyranuronic acid 2-sulfate and 2-deoxy-2-sulfamino-D-glucopyranose 6-sulfate, with a molecular weight range of 5,000-30,000. Purification employing the affinity of Heparin for various proteins is generally accomplished by covalently linking the Heparin to a support matrix, e.g., Heparin Sepharose® CL-6B consists of heparin coupled to Sepharose® CL-6B matrix by the cyanogen bromide method.

Heparin Chromatography is based upon two chief modes of interaction with proteins: (1) as an affinity ligand and (2) as a cation exchanger with the high content of anionic sulfate groups. However, for individual proteins, the exact nature of interaction with the Heparin is often by a combination of affinity and ion exchange interactions, and the exact nature of such interactions cannot be predicted accurately.

Heparin Chromatography has been applied to the purification of a wide variety of proteins (Affinity Chromatography, Principles and Methods; Pharmacia Publication 18-1022029): These include enzymes (mast cell proteases, lipoprotein lipase, coagulation enzymes, superoxide dismutase); serine protease inhibitors (antithrombin III, protease nexins); growth factors (fibroblast growth factor, Schwann cell growth factor, endothelial cell growth factor); extracellular matrix proteins (fibronectin, vitronectin, laminin, thrombospondin, collagens); nucleic acid-binding proteins (initiation factors, restriction endonucleases, DNA ligase, DNA and RNA polymerase); hormone receptors; (estrogen and androgen receptors) and lipoproteins.

One object of the present invention is to provide a suitable chromatographic step in an overall purification strategy of erythropoietins. As such, Heparin Chromatography can be employed as a first step or as a subsequent step in a multistep procedure. Heparin Chromatography provides a suitable purification step for selective removal of contaminants not duplicated by other purification processes described in the art, and, thereby offers an important alternative purification step in the preparation of homogeneous erythropoietins. The source of erythropoietin may be from a number of sources, including an organism (e.g. human urinary erythropoietin), from cell culture fluid following the introduction of an intact erythropoietin gene into mammalian cells, or from cell culture fluid following the activation of the endogenous erythropoietin gene in human cells.

Heparin Chromatography of the present invention is particularly suited to being incorporated into an overall purification scheme directed to obtaining homogeneous preparations of erythropoietin. Generally, to be commercially viable, such a process consists of a sequence of steps that are easily scaleable with yields that are able to meet pharmaceutical production requirements. The Heparin Chromatography step described herein is able to be incorporated into a large-scale purification scheme with high recovery for the purification of erythropoietins. A feature of the present invention is the ability of Heparin Chromatography to be incorporated either as a suitable first chromatographic step in an overall purification strategy of erythropoietins, or as a subsequent step for use at any position in a multistep process.

Many chromatographic steps are not well suited to be employed as a first chromatographic step. A first chromatographic step must be able to handle the bulk amount of nutrients and proteins initially present in a biological or culture fluid. A suitable first chromatographic step provides reasonable purification of the protein with high recovery of the desired protein and yet has adequate capacity for interaction with the load of bulk protein and media substituents. For instance, Heparin Chromatography served as a convenient and suitable first purification step independent of culture conditions, e.g., growth in fermenters or shake flasks, as anchorage-dependent or anchorage-independent cells, with or without serum proteins. The present Heparin Chromatography step has been found to meet these criteria as a suitable first step in the purification of erythropoietins.

Heparin Chromatography is also well suited for incorporation as a subsequent step for further purification of erythropoietins after initial fractionation by a number of purification steps, including but not limited to chromatography by ion exchange, hydrophobic interaction, size exclusion, or affinity, by fractionation by salt or alcohol precipitation, by preparative gel electrophoresis, or by preparative isoelectric focusing. Incorporation of Heparin Chromatography into an overall purification scheme can also enhance the effectiveness of later purification steps by the selective removal of contaminants that are not particularly well separated by later purification steps.

Heparin Chromatography offers a useful purification step for obtaining biologically active erythropoietins from biological or culture fluids with high recovery. In addition, Heparin Chromatography can be carried out with only aqueous solvents representing a further advantage as appreciated by those skilled in the art. Use of only aqueous solvents is advantageous in that the purification step can be designed such that the protein is not subjected to denaturants, organic solvents, or acids, that may be detrimental to recovering structurally intact and biologically active protein. Further, Heparin Chromatography can be run in very gentle conditions, e.g., at neutral pH, and/or at low salt concentrations. This property is particularly valuable in that erythropoietin is sensitive to aggregation or desialation at low or high pH and high salt conditions (Endo, Y., et al., J. of Biochem. 112, 700-706 (1992)). Therefore this technique offers very gentle aqueous conditions which serve to prevent loss of biological activity during chromatography of the erythropoietin molecules.

The object of the present invention relates to a chromatographic step in the purification of erythropoietin(s) which comprises: a) contacting a biological fluid containing erythropoietin with a heparin affinity support under conditions such that said erythropoietin molecules bind to said heparin affinity support; b) eluting said erythropoietin molecules with an elution buffer; and c) collecting said erythropoietin. The aforementioned chromatographic step can be used as either a first chromatographic step in the purification of erythropoietin or for inclusion in any point in a multistep process. The chromatographic step is particularly useful for removing contaminating proteins of erythropoietins, such as serum proteins, particularly, but not limited to those selected from the group of calf serum, bovine serum, fetal bovine serum, serum albumin, and transferrin. Within the scope of the present invention for the purification of erythropoietin(s) includes a chromatographic method whereby the contacted biological fluid is a culture fluid.

The present invention also relates to a heparin chromatographic step for inclusion in any point in a multistep process in the purification of erythropoietin(s), such as, but not limited to, a multistep process which additionally includes one or more chromatographic steps selected from the group of ion exchange, hydrophobic interaction, size exclusion, reverse phase, or affinity chromatography.

The invention further relates to a chromatographic step in the purification of erythropoietin(s) that can further comprise that the contacting biological fluid is clarified, such as performed by, but not limited to, centrifugation. Independently, the biological fluids can be centrifuged, filtered, ultrafiltered, dialyzed, or a combination of these procedures. When biological fluid is ultrafiltered, it can be performed on, but not limited to, a 5,000-100,000 D cutoff membrane or 10,000-30,000 D cutoff membrane. When a biological fluid is filtered it can be obtained optionally, but not limited to, a 45 micron membrane.

In the present invention, binding of said erythropoietin molecules can, but not limited to, occur in an equilibration buffer. Such buffers include, but or not limited to, 4-(2-hydroxyethyl)-1-piperazinoethane sulfonic acid buffer (MES), tris(hydroxymethyl)aminomethane (Tris), or phosphate buffer, or any other buffer that provides a suitable buffer for binding erythropoietins. In addition, binding of said erythropoietin molecules can occur, but not limited to, at around a pH of 4.9 to 8.0, and preferrably at around a pH of 5.9 to 7.5, and more preferrably at a pH of 6.2 or 7.

In addition, the chromatographic steps of the present invention comprises eluting said erythropoietins by varying the elution buffer, optionally, but not limited to, by increasing the salt concentration in the buffer, washing the column with additional buffer, or varying the operating pH of the elution buffer. The salt concentration of the elution buffer can be adjusted by, but not limited to, the addition of alkali halides, such as sodium chloride (NaCl). A further embodiment of the present invention includes opptionally adding to any of the buffers an anti-adsorbent, such as, but not limited to, Tween.

The chromatographic steps of the present invention further include exchanging the buffer of the medium containing erythropoietin, such as, but not limited to a biological fluid or collected sample. Such buffer exchange procedures include, but are not limited to, the use of tangential flow systems, hollow fibers, spiral wound filters, stirred cells systems, dialysis, diafiltration, filtration step, or by conventional chromatography. When buffer exchange is performed by a dialysis or diafiltration a membrane, one can optionally use, but not limited to, a 5,000 to 10,000 or 10,000 to 30,000 molecular weight membrane. Buffer exchange can be performed at a chosen pH and buffer salt concentrations, such as, but not limited to, those pH's between a pH of 6 and 8, 5.9 and 7.5. and 6.2 to 7.0, and against a buffer of 0 to 200 mM salt or more preferably 0 to 10 mM salt.

Another embodiment of the present invention anticipates the use of a number heparin affinity supports that are commerically available supports, such as, but not limited to Heparin Sepharose CL-GB®, Heparin Sepharose 6 Fast Flow®, Hitrap Heparin®, Affigel Heparin®, Heparin Agarose®, Heparin Sepharose®, Heparin Hyper D®, and Heparin Agarose 6XL®.

### BACKGROUND OF THE INVENTION

Erythropoietins produced by mammalian cells in vitro or in vivo are glycoproteins having a mature amino acid sequence of 165 amino acids and containing three N-linked and one O-linked oligosaccharide chains. The N-linked oligosaccharide chains occur at asparagine residues 24, 38, and 83, while the O-linked oligosaccharide chain occurs at serine residue 126. Lai et al. J. Biol. Chem. 261, 3116 (1986); Broudy et al. Arch. Biochem. Biophys. 265, 329 (1988). The 165 amino acid coding region and the processed leader sequence of erythropoietin is known in part from the isolation of genomic and cDNA clones of the human erythropoietin gene. Jacobs et al. Nature 313, 806 (1985); Lin, F.K., et al. Proc. Natl. Acad. Sci. U.S.A. 82, 7580 (1985).

A large number of scientific papers, patents, and patent applications deal with isolation of erythropoietin. A brief summary of related references indicates that a wide variety of chromatographic steps have been applied to the isolation of erythropoietins. The following references, indicated below, are a listing of sources and procedures, yet no attempt has been made to describe in detail the operation of each chromatographic step. Further, the following references attempt only to indicate the state of the art as a whole, and are not to be taken as an exhaustive list of all of what is known in the art.

Use of both anion and cation exchange chromatography for purification of erythropoietins has been described in the scientific literature: (1) (Mono Q® ) Broudy, V.C. (1988) Arch. of Biochem. and Biophys. 265, 329-336; (2) (DEAE Sephacel® ) Ghanem, A. B. et al. (1994) Preparative Biochemistry 24, 127-142; Quelle, F.W. et al. (1989) Blood, 74, 652-657; (3) (DEAE Agarose®) Miyake, T. et al. (1977) J. Biol. Chem 252, 5558-5564; (4) (S-Sepharose®) Fibi, M.R., et al (1991) Blood 77. 1203-1210; (5) (Sulfoethyl Sephadex®) Goldwasser, E. and Kung, C.K.H. (1971) PNAS 68, 697-698; and (6) (Sulfopropyl Sephadex®) Miyake, T. et al. (1977) J. Biol. Chem. 252, 5558-5564.

Particular use of weakly basic anion exchange resins, such as DEAE, as a first chromatographic step is well described in the patent literature: (1) US 4397840 (Priority application 81JP-0034727); and (2) JP 56016496A (Priority Application 79JP-0092999). Use of more strongly basic anion exchange resins for isolating erythropoietin isoforms using Q-Sepharose® is described in the patent literature: (1) EP 4282267A2, WO91058677, and EP 428267A (Priority 89US-0421444). Use of strong ion exchange resins as a general method to separate desired proteins from impurities is published in the patent literature: EP 313343A, EP 391934A, and WO8903840A (Priority Application 87US-0111886).

GB-A-886 712 discloses methods for the preparation of erythropoietic factor concentrates using an insoluble polymer containg ion exchange groups.

Affinity chromatography using Cibacron Blue dyes has been described in the scientific literature in the purification of erythropoietins: (1)(CM Affi-Gel Blue®) Krystal, G. et al. (1984) Brit. J. of Hematology 58, 533-64; and (2) (CM Affi-Gel Blue®) Broudy, V. et. (1988), Arch of Biochem and Bioph, 265, 329-336; (3) (CM Affi-Gel Blue®) Krystal, G. et al. (1986), Blood, 67, 71-79; (4) (CM Affi-Gel Blue®) Yanagi, H., et al (1989), DNA, 8, 419-427; (5) (Blue Trisacryl M®) Fibi, M.R. et al (1991) Blood, 77, 1203-1210.

Use of anthraquinones, like Cibacron Blue dyes, is also known in the patent literature: (1) JP 59065021A (Priority Application 82JP-0174678); (2) (bound carboxymethyl groups and Cibacron Blue F3GA® as CM Affi-Gel Blue®) JP01165393A (Priority Application 87JP-0324910).

Use of size exclusion chromatography for purification of erythropoietins has been described in the scientific and patent literature: (1) (TSK G3000SW® ) Yanagi, H. et al. (1989) DNA 8, 419-427; (2) Fibi, M.R., et al. (1991) Blood 77, 1203-1210; (3) (Sephadex G-100®) Goto, M. et al. (1988) Biotechnology 6, 67-70; Yanagawa, S. et al. (1984) J. Biol. Chem. 259, 2707-2701; Miyake T. et al. (1977) J. Biol. Chem 252, 5558-5564; (4) JP 01165393A (Priority Application 87JP-0324910)

Use of hydroxyapatite to purify erythropoietins has been described in the scientific and patent literature: Krystal, G. et al (1986) Blood 67, 71-79; Yanagi, H. et al. (1989) DNA 8, 419-427; (BioRad HPHT®) Goto, M. et al. (1988) Biotechnology 6, 67-70; (calcium phosphate gel) Goldwasser, E. and Kung, C.K.H. (1971) PNAS 68, 697-698; (BioRad BioGel HT®) Miyake, T. et al. (1977) J. Biol. Chem. 252, 5558-5564; JP 01165393A (Priority Application 87JP-0324910). Separation of impurities of proteins containing initiator methionines (Met-proteins) using hydroxyapatite, especially in an HPLC mode, or in a combination of steps, is given in US 4798886, WO8602068A, EP176299A, and EP 239311A (Priority Applications B4WO-JP00460, 86JP-0066399 and 87JP-0066940); US 5256769 and EP 176299B.

General use of immobilized antibodies for immunoaffinity chromatography of erythropoietins is known in the scientific literature: Miyazaki, H. et al. (1988) J. of Immunological Methods, 113, 261-267; Goto, M. et al. (1988) Biotechnology 6, 67-70; Yanagawa, s. et al., (1984) Preparative Biochemistry 24, 127-142; Ghanem, A.B. et al. (1994) Preparative Biochemistry 24, 127-142; Wojchowski, D.M. et al. (1987) Biochim. Biophys. Acta 913, 170-177.

General use of immobilized antibodies for immunoaffinity chromatography are also known in the patent literature: EP 249932A (Priority Application 86JP-0139142), and more specifically to erythropoietin in US 4558006, US 5106760 and EP 116446B1 (Priority Application 83US-0463724); JP 56108798A (Priority Application 80JP-0011685); JP 4117400A, JP 59116297A and JP 94089040B2 (Priority Application B2JP-0231539), JP 940866480B2, JP 04117400 A, US 4465624 (Priority Application 83JP-0026399). US 4831118 (Priority Application 87US-0083670), US 4568488 (Priority Application 84US-0570075), and US 4558005.

Use of reverse-phase high pressure liquid chromatography (RP-HPLC) as a step in the overall purification of erythropoietins is known in both the scientific and patent literature: (1) (DEAE-C4 RP-HPLC - Con A Agarose) Quelle, F. W. et al. (1989) Blood, 74, 652-657; (2) (Blue Trisacryl M® - S-Sepharose® - C8 RP-HPLC - Size exclusion) Fibi, M.R., et al. (1991) Blood, 77, 1203-1210; (3) (RP-HPLC - molecular sieve chromatography) JP 62036400A (Priority Application 85JP-0177337).

RP-HPLC has also been described as a final chromatographic step in the scientific and patent literature: Jacobs, K. et al. (1989), Nature 313, 806-810; (cation exchange chromatography - RP-HPLC) Broudy, V. et al. (1988), Arch of Biochem and Bioph, 265, 329-336; Krystal, G. et al. (1986), Blood, (DEAF-Agarose® - Sulphopropyl-Sephadex® - gel filtration - hydroxyapatite - RP-HPLC) EP 209539B1 and US 5322837 (Priority Application 85US-0690853); US Patent 4, 677, 016, EP 228452A, WO 8607594A (Priority Application 86US-0872152).

A number of additional steps for the purification of erythropoietins are known in the scientific and patent literature: (1) (Lectin affinity chromatography) Krystal, G. et al. (1986) Blood 67, 71-79 (Wheat Germ); Quelle, F.W. et al. (1989) Blood 74, 652-657; EP 358463A (Priority application 88ZA-0006645)(2) (Chromatofocusing) Krystal, G. et al. (1986) Blood, 67, 71-79. PBE94; (3) (Preparative SDS-PAGE) Krystal, G. et al (1986) Blood, 67, 71-79; and (4) (Methylated albumin-Kieselguhr); Goldwasser. E. and Kung, C.K.H. (1971) PNAS 68, 697-698; (5) (absorption to the support of porous polystyrene, chitosan or diatomaceous earth) US 4303650 having as priority application 79JP-01306677; (6) (Hydrophobic Interaction Chromatography) Lee Haung et al. (1980), Blood, 56, 620-624; Wojchowski et al. (1987) Biochim. et Biophys. 913, 170-178.

Among the extensive techniques reported in the scientific and patent literature for the purification of erythropoietin, none of the aforementioned references employ Heparin Chromatography. The present invention therefore offers a novel and advantageous purification step in the purification of erythropoietins.

### DESCRIPTION OF THE DRAWINGS

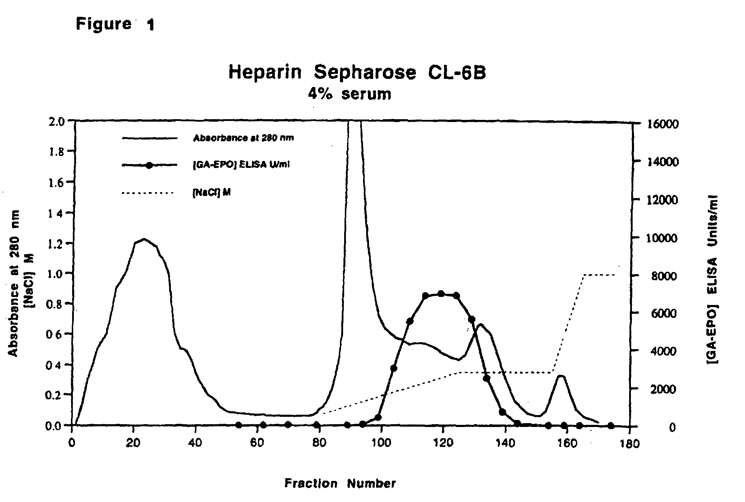

### FIG 1. Heparin-Sepharose® Chromatography of GA-EPO From 4% Serum

Figure 1 is a chromatogram showing the Heparin-Sepharose® CL-6B fractionation of culture supernatant of a cell line expressing GA-EPO. The cells are propagated as an anchorage-dependent culture in a Cell Cube (Costar) in 4% fetal bovine serum-containing medium. The ultrafiltered, diafiltered culture supernatant described in Example 1 is separated on Heparin-Sepharose® CL-6B at pH 6.2, and GA-EPO content of the fractions is determined by ELISA (_o_). The __ line represents absorbance at 280 nm. The --- line represents the NaCl gradient.

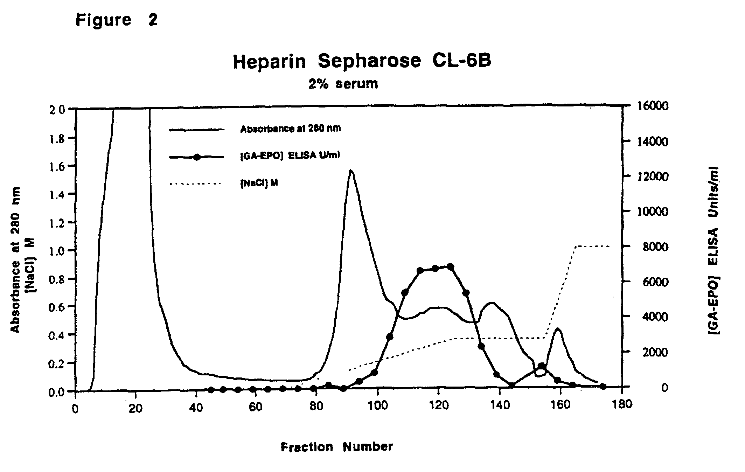

### FIG 2. Heparin-Sepharose® Chromatography of GA-EPO From 2% Serum

Figure 2 is a chromatogram showing the Heparin-Sepharose® CL-6B fractionation of GA-EPO derived from culture supernatant of a cell line expressing GA-EPO. The cells are propagated as an anchorage-dependent culture in a CellCube (Costar) in 2% fetal bovine serum-containing medium. The ultrafiltered, diafiltered culture supernatant of Example 2 is separated on Heparin-Sepharose® CL-6B at pH 6.2, and GA-EPO content of the fractions is determined by ELISA (_o_). The (__) line represents absorbance at 280 nm. The (---) line represents the NaCl gradient.

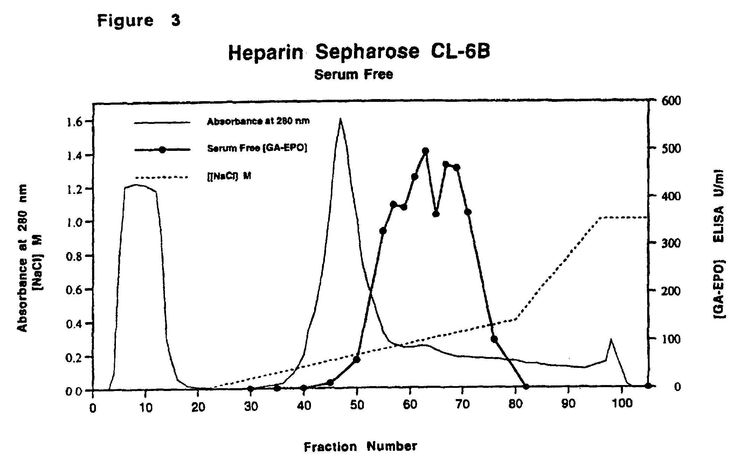

### FIG 3. Heparin-Sepharose® Chromatography of GA-EPO From Serum Free Medium

Figure 3 is a chromatogram showing the Heparin-Sepharose® CL-6B fractionation of GA-EPO derived from culture supernatant of a cell line expressing GA-EPO. The cells are propagated in suspension in spinner flasks in serum-free medium. Ultrafiltered, diafiltered culture supernatant of Example 3 is separated on Heparin-Sepharose® CL-6B at pH 6.2, and GA-EPO content of the fractions is determined by ELISA (_o_). The (__)line represents absorbance at 280 nm. The (---) line represents the NaCl gradient.

### FIG 4. Heparin-Sepharose® 6 Fast Flow Chromatography of GA-EPO From 4% Serum

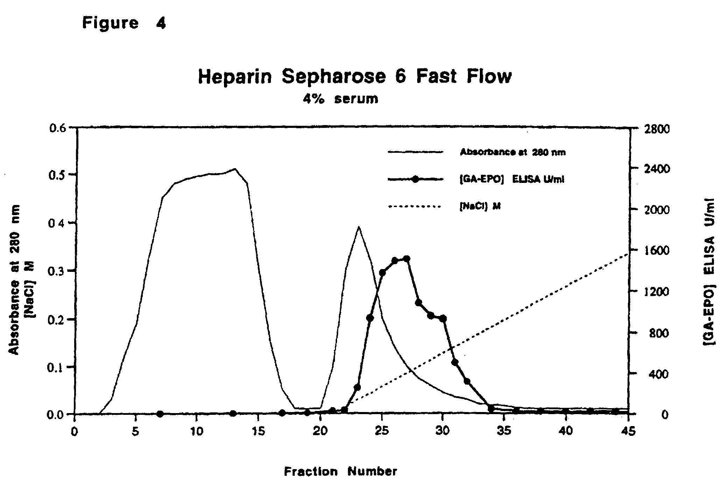

Figure 4 is a chromatogram showing the Heparin-Sepharose® 6 Fast Flow fractionation of GA-EPO derived from culture supernatant of a cell line expressing GA-EPO. The cells are propagated as an anchorage-dependent culture in a Cell Cube (Costar) in 4% fetal bovine serum-containing medium. The ultrafiltered, diafiltered culture supernatant of Example 4 is separated on Heparin-Sepharose® 6 Fast Flow at pH 7.0, and GA-EPO content of the fractions is determined by ELISA (_o_). The (__) line represents absorbance at 280 nm. The (---) line represents the NaCl gradient.

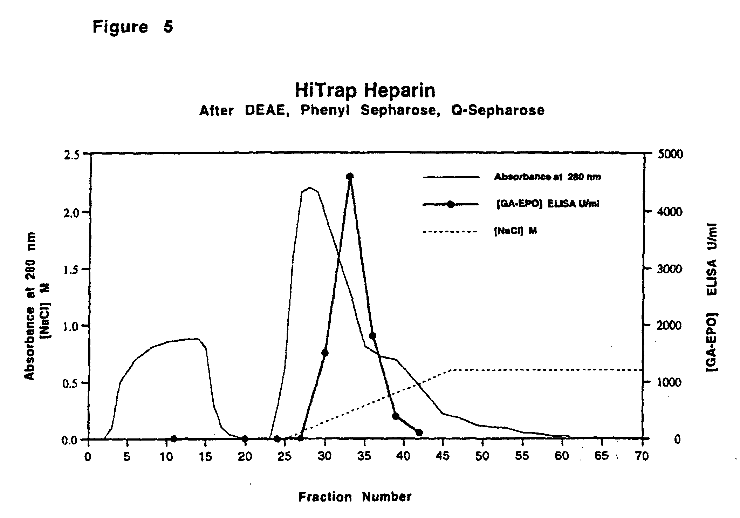

### FIG 5. HiTrap Heparin Chromatography of GA-EPO from 2% Serum After Initial Purification by Ion Exchange and Hydrophobic Interaction Chromatography

Figure 5 is a chromatogram showing the HiTrap Heparin® (Pharmacia) fractionation of GA-EPO derived from culture supernatant of a cell line expressing GA-EPO. The cells are propagated in roller bottles in 2% calf serum-containing culture medium. Initial fractionation by Ion Exchange Chromatography using Pharmacia Streamline® DEAE is followed by Hydrophobic Interaction Chromatography on Phenyl Sepharose® Fast Flow (Pharmacia) and Ion Exchange Chromatography on Q-Sepharose® Fast Flow (Pharmacia). The partially purified material eluted from the Q-Sepharose® resin is dialyzed and is separated on HiTrap Heparin® at pH 6.2, and GA-EPO content of the fractions is determined by ELISA (_o_). The (―) line represents absorbance at 280 nm. The (---) line represents the NaCl gradient.

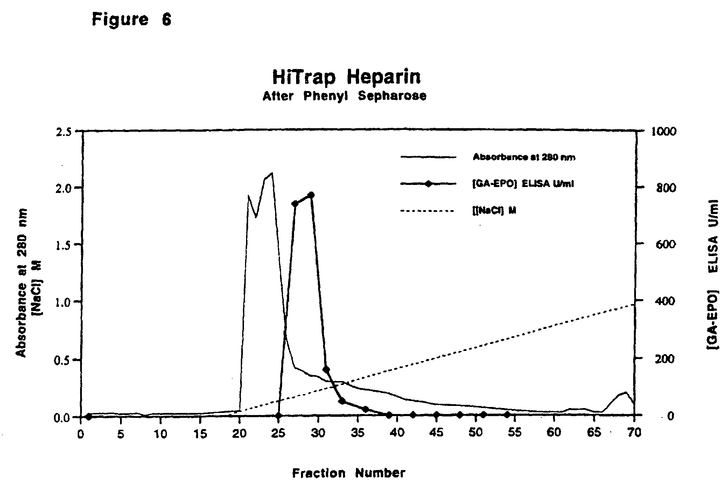

### FIG 6. HiTrap Heparin® Chromatography of GA-EPO From 2% Serum After Initial Purification by Hydrophobic Interaction Chromatography

Figure 6 is a chromatogram showing the HiTrap Heparin® (Pharmacia) fractionation of GA-EPO derived from culture supernatant of a cell line expressing GA-EPO. The cells are propagated in roller bottles in 2% calf serum-containing culture medium. Initial fractionation is by Hydrophobic Interaction Chromatography on Phenyl Sepharose® 6 Fast Flow (Pharmacia). The partially purified material eluted from this resin is dialyzed and is separated on HiTrap Heparin® at pH 5.2, and GA-EPO content of the fractions is determined by ELISA (_o_). The (―) line represents absorbance at 280 nm. The (---) line represents the NaCl gradient.

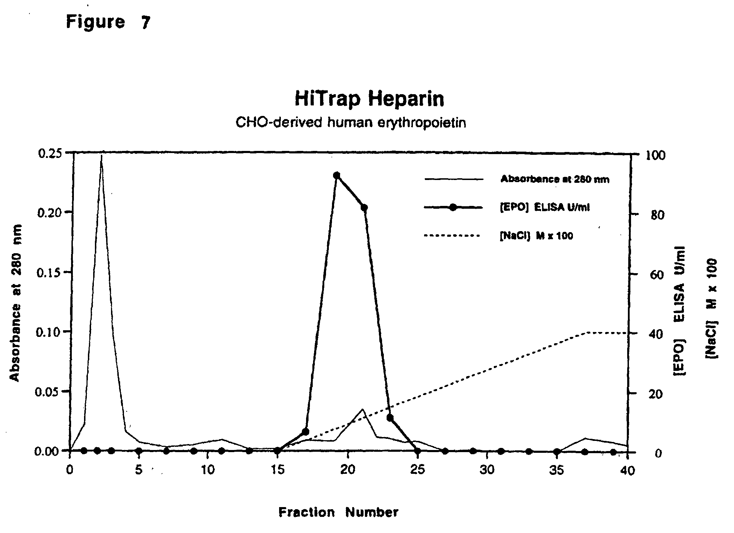

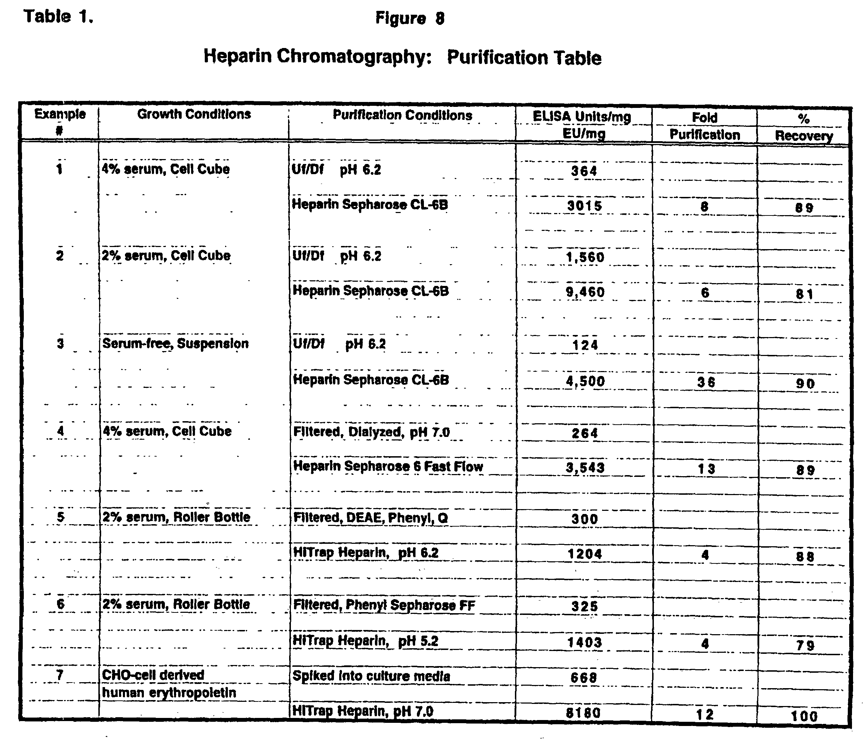

### FIG 7. HiTrap Heparin Chromatography of Erythropoietin Derived From CHO Cells

Figure 7 is a chromatogram showing the HiTrap Heparin® (Pharmacia) fractionation of Chinese Hamster Ovary (CHO) cell-derived erythropoietin (R&D Systems) spiked into diafiltered, unconditioned medium containing 2% calf serum. The spiked, diafiltered medium is separated on HiTrap Heparin® at pH 7.0, and the erythropoietin content of the fractions is determined by ELISA (-_-). The solid line (―) represents absorbance at 280 nm. The broken line (-----) represents the NaCl gradient.

### DESCRIPTION OF THE TABLE

### TABLE 1. Heparin Chromatography: Purification Table

Table 1 compiles some of the data obtained using Heparin Chromatography as a first or subsequent chromatographic step. The examples shown cover a wide variety of culture conditions including anchorage-dependent and anchorage-independent cells, and with or without either fetal bovine serum or calf serum at various concentrations in the medium. Similarly, the examples demonstrate a wide variety of chromatographic conditions. Given sequentially in the table are Example # (column 1), Growth Conditions (column 2), Purification Conditions (column 3), ELISA Units/mg (specific activity expressed as EU/mg; column 4), Fold Purification (column 5), and percent (%) recovery (column 6)

### DETAILED DESCRIPTION

Erythropoietin regulates the growth and terminal maturation of erythroid progenitor cells to mature red blood cells. Erythropoietin is produced in the human adult kidney, erythroblastoid cells, and in fetal liver cells. Patients with impaired renal function have a decreased production of erythropoietin, leading to severe anemia. The use of purified natural and recombinant erythropoietin has been an effective treatment for patients with chronic anemia (Canaud, B. (1990), Am. J. Kidney Dis. 15, 169-175; Faulds, D. et al. (1989), Drugs 38, 863-899; Winearles, C. G. et al. (1986) Lancet 1175-1178).

Erythropoietin is actually comprised of a vast number of chemically distinct species due to a complex array of post-translational modifications, particularly glycosylation. The erythropoietins described, herein, have both of the following features in common: (1) they have an amino acid sequence that sufficiently resembles that encoded by the human erythropoietin gene; and (2) they possess in vivo biological properties causing an increase in the production of reticulocytes and red blood cells.

As previously mentioned, both human urinary and mammalian cell produced recombinant erythropoietins are glycoproteins having a mature amino acid sequence of 165 amino acids and containing three N-linked and one O-linked oligosaccharide chains. The N-linked oligosaccharide chains occur at asparagine residues 24, 38, and 83, while the O-linked oligosaccharide chain occurs at serine residue 126. Lai et al. J. Biol. Chem. 261, 3116 (1986); Broudy et al. Arch. Biochem. Biophys. 265, 329 (1988). Erythropoietin (EPO) has an apparent molecular weight that is varied depending on gel electrophoretic conditions (see for instance Broudy, V., et al., Arch of Biochem Biophys. 265, 329-336 (1998); Krystal, G., et al. Blood 67(1), 71-79 (1986); Yanagi, H., et al., DNA 8(6), 419-427 (1989). The actual molecular weight is variable and spans a range of sizes due to a diversity of carbohydrate structures attached to the protein portion of the molecule. This diversity is in part dependent upon the in vivo or in vitro cell type producing the protein.

The purified erythropoietins of the present invention may be any of the known erythropoietins of biological and/or pharmaceutical interest. As used herein, the term "erythropoietin" or "erythropoietin product" is intended to include (1) erythropoietin isolated from an in vivo source, for example human urine as described by Miyake, T., et al. (1977) J. of Biol. Chem. 252(15) 5558-5564, (2) that produced from activation of the erythropoietin gene in human-derived cells, such as that described in WO 94/12650 (also indicated as GA-EPO), (3) that produced from the introduction of an erythropoietin gene into a human host cell, for example as described by Yanagi, H., et al. (1989) DNA 8(6), 419-427, and (4) that produced from the introduction of an erythropoietin gene into a non-human host cell, such as that described in US Patent 4, 703, 008.

"Producing cells" may be cells of eukaryotic origin, preferably of vertebrate origin, and more preferably of mammalian origin, and most preferably from human origin. Producing cells are capable, upon cultivation in a suitable medium, of producing the desired erythropoietin in a recoverable amount. Representative examples of these cells include fibroblasts, keratinocytes, epithelial cells (e.g., mammary epithelial cells, intestinal epithelial cells), endothelial cells, glial cells, neural cells, cells found in the blood (e.g., lymphocytes, bone marrow cells), muscle cells, and precursors of these somatic cell types. As mentioned, these cells are more preferably of mammalian origin (e.g., mouse, rat, rabbit, cat, dog, pig, cow, bird, sheep, goat, horse, monkey, human) and most preferably they are of primate or human origin. Producing cells also encompasses transfected primary, secondary, and immortalized cells of vertebrate origin, particularly of mammalian origin and most preferably of primate or human origin transfected with exogenous genetic material that directly or indirectly causes the cells to produce recoverable amounts of erythropoietin.

The Heparin Chromatographic process of the present invention is directed to isolation of erythropoietin from "biological fluids". As used herein, "biological fluids" incorporates erythropoietins isolated from inside a cell, such as from the cytoplasm or from inclusion bodies or vacuoles, or isolated from the medium, such as when the cell secretes the erythropoietin. The secreted molecules may be found and then isolated from a biological fluid, such as blood, milk, ascites or urine, or a cell culture fluid.

In instances when cells are cultured, the erythropoietin is secreted into a "culture fluid". The Heparin Chromatography process for isolation of erythropoietin is particularly suited to the recovery of erythropoietin contained in a culture fluid of an erythropoietin producing cell. As used herein, the term culture fluid is preferably intended to refer to.any fluid used or derived from cultured cells capable of producing erythropoietin.

Preferably, the biological or culture fluid used for purification of the erythropoietin has been separated from cells and cell debris by filtration, ultrafiltration, centrifugation, or by other techniques known by those skilled in the art. Moreover, the term "clarified fluid" is used to indicate a biological or culture fluid that has been separated from cells, cell debris, or particulate matter by centrifugation, filtration, ultrafiltration, or similar technique known in the art. Since erythropoietin molecules are most often purified from a "clarified fluid" it is advantageous to separate the cell and particulate debris from the medium whether it is a biological or culture fluid. This process can be done by a number of steps, including centrifugation and filtration, including ultrafiltration (Uf), as well known in the art. The term "clarified fluid" may also include erythropoietin containing fluids that have been subjected to a prior chromatographic or purification step in which the cell and cell debris as well as other cell and media contaminants have been removed from the sample.

Often it is desired to "buffer exchange" the medium containing the erythropoietin with a known laboratory buffer. Such a step can be done independently as a separate step or in conjunction with other steps. "Buffer exchange" can be conducted by a number of procedures well known to those skilled in the art, including, but not limited to, tangential flow systems, hollow fibers, spiral wound filters, or a stirred cell system, or by conventional chromatography as known in the art. A more preferred process as used herein is by diafiltration (Df). In such cases, preferably, the membrane is a polysulfone membrane or regenerated cellulose membrane of the types commercially available, e.g. from Millipore or Amicon Inc. Also preferred for use in a filtration step are those membranes having a cutoff of about 5,000 to 100,000 molecular weight, and more preferably a cutoff of about 10,000 to 30,000 molecular weight. The retentate is then optionally submitted to dialysis and/or diafiltration according to procedures known in the art, preferably in the same buffer having a pH between 6 and 8. The salt is present preferably from 0 to 200 mM and still more preferably, the salt is 0 to 10 mM. The pH is preferably from 4.9 to 8.0, more preferably 5.9 to 7.5, and most preferably about pH 6.2 to 7.0.

Generally, the major contaminating proteins in mammalian cell culture fluids are derived from serum or proteins added to the medium. This is in part because of the dependence of many cell types on a significant amount of serum being present. Commercial cell media are often acquired with, or have had added, either calf serum, bovine serum, fetal bovine serum, or serum albumin or other purified or partially purified serum proteins such as transferrin or combinations thereof, from which the desired protein must be purified. Therefore, it is often desirable that the first step of the purification process is directed to removal of serum components.

The Heparin Chromatography process is not limited to the first step of a purification process. It is suitable for inclusion in a purification process at any point in a multistep process. Preparation of partially purified erythropoietins by any partial fractionation procedure or chromatographic procedure including, but not limited to, ion exchange, hydrophobic interaction, size exclusion, reverse phase or affinity, can be performed prior to Heparin Chromatography.

A "heparin affinity support" refers to any known chromatographic support bearing heparin molecules wherein the heparin is stably bound to the resin support and used for Heparin Chromatography. Heparin affinity supports are available from a number of commercial suppliers. Commercially available heparin affinity supports include, but are not limited to, Heparin Sepharose® CL-6B, Heparin Sepharose® 6 Fast Flow, HiTrap Heparin® (Pharmacia); Affigel Heparin® (BioRad); Heparin Agarose®, Heparin Sepharose® Type I and Type II, Type ITS, Type IIS (Sigma); AF-Heparin Toyopearl 650®. Heparin-650M® (TosoHaas); Cellufine Heparin® (Amicon); Heparin Hyper D® (Biosepra); Heparin Agarose 6xL® (types I, II, and III) (American International Chemical). A preferable solid matrix is agarose-based, most preferably Sepharose® or Sepharose® derivatives in bead form. Other solid matrices such as celluloses or polyacrylamides may also be used.

Heparin Chromatography may be carried out either batchwise or by column elution. Preferably the heparin support is in a column chromatographic system. When used in column chromatographic systems, the heparin affinity support is used preferably between 0oC and 30oC, more preferably between 4oC and 25oC, and most preferably at about 4oC. Preferably the Heparin resin is generally equilibrated, prepared and maintained according to the manufacturer's specifications (e.g., Heparin Sepharose® CL6B is prepared as described in the Pharmacia Instruction Manual 71-7078-00).

Binding of erythropoietin molecules to a heparin support is accomplished by first equilibrating the support in an "equilibration buffer." The "equilibration buffer" is the hydrous state in which the support is placed for binding the erythropoietin molecules when contacted with a biological or culture fluid. Binding of erythropoietin to the column is accomplished at around a pH of 4.9 to 8.0, and more preferably around pH 5.9 to 7.5, and most preferably around pH 6.2 or 7.0. A number of buffers are suitable for use with Heparin supports. Preferred buffers include MES (2-[N-Morpholino]ethanesulfonic acid), Tris (tris[Hydroxymethyl]aminomethane) or phosphate. The preferably selected concentration for the equilibrating buffer is about 20 mM, with a preferred conductivity of about 2 mS/cm.

Prior to eluting bound erythropoietin from the Heparin support, contaminants may be eluted by washing the column with additional equilibration buffer, or with the equilibration buffer containing added salts, buffers, or by varying the operating pH or in combinations thereof. For example, binding of the erythropoietin may be accomplished at pH 6.2, and some contaminants may be eluted at pH 7.4 without loss of erythropoietin. Altering the buffer system, by manipulation of its components, thereby, may be used to achieve a suitable purification of erythropoietin molecules. For instance, elution of erythropoietin from the Heparin support can be accomplished by increasing the salt concentration present in the elution buffer. Preferred salts are the alkali halides, and most preferred is the use of NaCl.

Optionally, the buffers for equilibrating or eluting erythropoietin contain an "anti-adsorbent." An "anti-adsorbent" is any substance that reduces adsorption of erythropoietin to container surfaces or to other proteins, or to itself (e.g., aggregation). Preferred examples of anti-adsorbents are the polyoxyethylene sorbitan derivatives of fatty acids known as "Tween", with "Tween 20®" (polyoxyethylene sorbitan monolaurate) the currently preferred surfactant. The surfactant, if present, is preferably employed at a concentration of about 0.01 to 0.5% and more preferably about 0.1% (w/w).

The present Heparin purification step can be incorporated into an overall purification strategy for erythropoietin. For instance, the present step may be employed with traditional chromatographic steps, such as ion exchange, gel permeation chromatography, and reverse phase HPLC.

Ion exchange steps in combination with the Heparin step (either before or after Heparin Chromatography) include the use of anion exchange or cation exchange chromatography. Generally known commercially available anion exchange supports used in the purification of erythropoietin bear quaternary ammonium functional groups. Preferred matrices for use in the present process are agarose or cellulose based matrices, such as microcrystalline cellulose or cross-linked agaroses. Also particularly preferred are those matrices bearing diethyl aminoethyl, triethyl aminomethyl, or trimethyl aminomethyl functional groups. A particularly preferred anion exchange matrix is trimethyl aminomethyl cross linked agarose, which is commercially available, e.g. Q-Sepharose Fast Flow® (Pharmacia). Generally known commercially available cation exchange supports that may be used in the purification of erythropoietins bear acidic functionalities, including carboxy and sulfonic acids. Matrices containing the cation functionalities include various forms of celluloses and polystyrene based matrices. For example, weak cation exchangers known in the art include, but are not limited to, Carboxymethyl-Cellulose®, Carboxymethyl-Sephadex®, and Carboxymethyl-Sepharose®. Strong cation exchangers known in the art include, but are not limited to, sulfonated polystyrenes (AG 50W®, Bio-Rex 70®), sulfonated celluloses (SP-Sephadex®), and sulfonated Sepharoses (S-Sepharose®).

The chromatographic step involving these matrices is most preferably conducted as a column chromatography step which optionally can be performed at a temperature between 40 to 250 degrees centigrade. Generally, a salt is added to a washing or eluting buffer to increase the ionic strength of the application or equilibrating buffer. Any of the salts conventionally used may be employed for this purpose as can be readily determined by one skilled in the art, with NaCl being one of the most frequently and conveniently used salts.

A conventional gel filtration step can also be used in combination with the Heparin process step. Representative examples of these matrices are polydextrans cross linked with acrylamides, such as composite hydrophilic gels prepared by covalently cross linking allyl dextran with N,N'-methylene bisacrylamide and crosslinked cellulose gels. Commercially available crosslinked dextran-acrylamides are known under the trade name Sephacryl® and are available from Pharmacia. A preferred Sephacryl® gel is Sephacryl S-200 HR® . Examples of cross linked cellulose gels are those commercially available crosslinked porous cellulose gels, e.g. GLC 300® or GLC 1,000® that are available from Amicon Inc.

### EXAMPLES

The following examples are only illustrative and are not to be construed to limit the scope of the claimed invention. Starting material for purification of erythropoietins can be derived from biological fluids or from anchorage-dependent or anchorage-independent cells cultured in any suitable culture medium with or without various amounts of added bovine serum or other serum or non-serum derived components. Starting material may be produced in a variety of human or non-human cell types. Examples of immortalized human cell lines useful for erythropoietin production include, but are not limited to, HeLa cells and derivatives of HeLa cells (ATCC CCL 2, 2.1 and 2.2), MCF-7 breast cancer cells (ATCC HTB 22), K-562 leukemia cells (ATCC CCL 243), KB carcinoma cells (ATCC CCL 17), 2780AD ovarian carcinoma cells (Van der Blick, A.M. et al., Cancer Res, 48:5927-5932 (1988), Raji cells (ATCC CCL 86), Jurkat cells (ATCC TIB 152), Namalwa cells (ATCC CRL 1432), HL-60 cells (ATCC CCL 240), WiDr cells (ATCC CCL218), HT1080 cells (ATCC CCL 121), Daudi cells (ATCC CCL213), RPMI 8226 cells (ATCC CCL 155), U-937 cells (ATCC CRL 1593), Bowes Melanoma cells (ATCC CRL 9607), WI-38VA13 subline 2R4 cells (ATCC CCL 75.1), and MOLT-4 cells (ATCC CRL 1582), as well as heterohybridoma cells produced by fusion of human cells and cells of another species. Secondary human fibroblast strains, such as WI-38 (ATCC CCL 75) and MRC-5 (ATCC CCL 171) may be used. In addition, primary human cells may be used for erythropoietin production. Erythropoietin may be produced by activation of the erythropoietin gene in primary, secondary, or immortalized human cells, essentially as described in WO 94/12650, or by transfection of an erythropoietin gene into primary, secondary, or immortalized cells of human or non-human origin.

The illustrative examples show the versatility of Heparin Chromatography as a first or subsequent purification step. The following examples also demonstrate that Heparin Chromatography works well with a variety of commercial Heparin resins and also demonstrate the scalability of the step from analytical to preparative use. Examples 1-6, and 8-9 are performed using GA-EPO isolated from gene activated human cells, produced essentially as described in WO 94/12650. Example 7 is performed using erythropoietin from a commercially available source which was produced by expression of the human erythropoietin gene after transfection into CHO cells.

### EXAMPLE 1.

### Use of Heparin Chromatography as a First Chromatographic Step in a Purification Process

### I.1. Starting Material

Conditioned medium containing 4% fetal bovine serum is obtained from an adherent human cell line expressing GA-EPO propagated in a Cell Cube (Costar). The medium is clarified by centrifugation followed by filtration through a 0.45 micron Nalgene filter unit.
Ultrafiltration/diafiltration (Uf/Df) is performed using a Pellicon tangential flow system (Millipore) fitted with a 30,000 molecular weight cutoff membrane. The equilibration and dialysis buffer is 20 mM MES, pH 6.2, containing 0.5% Tween 20.

### I.2. Heparin Chromatography

Heparin Chromatography is carried out using Pharmacia's GradiFrac System at 4oC. A 280 ml column (14 x 5 cm) of Heparin Sepharose® CL-6B (Pharmacia) is equilibrated with 20 mM MES, pH 6.2, and containing 0.1% Tween 20, at a flow rate of 10 ml/min. Sample is applied to the column at 10 ml/min with a Pharmacia P 50 pump. The column is then washed with equilibration buffer until the absorbance at 280 nm stabilizes. Loosely bound protein is eluted by washing with one column volume (280 ml) of 20 mM MES, pH 6.2, containing 0.1% Tween 20 and 0.05 M NaCl while collecting 12 ml fractions. The wash is followed by a two column volume (560 ml) linear gradient from 0.05 M to 0.35 M NaCl in 20 mM MES, pH 6.2, containing 0.1% Tween 20, then washed with one column volume of 0.35 M NaCl in 20 mM MES, pH 6.2, containing 0.1% Tween 20. The elution buffer is then increased to 1.0 M NaCl in 20 mM MES, pH 6.2, containing 0.1% Tween 20 over one half column volume (140 ml) and then held in this buffer for one column volume (280 ml).

Total protein is monitored by absorbance at 280 nm and salt concentration is followed by an in-line conductivity monitor (Figure 1). The flow-through and eluant fractions are analyzed for GA-EPO content by ELISA (R & D Systems), SDS-PAGE, and Western Blot.

The Heparin Sepharose® column is regenerated by application of 560 ml of 20 mM Tris-Cl, pH 9.0, containing 4 M Urea and 1.4 M NaCl, followed by a wash with equilibration buffer until the conductivity and pH of the column effluent equals that of the equilibration buffer.

In one experiment, of the GA-EPO loaded onto the Heparin column, 89% was recovered in the elution pool. Use of this column resulted in removal of 90% of the contaminating protein (Table 1).

### EXAMPLE 2

### Use of Heparin Chromatography as a First Chromatographic Step in a Purification Process

### II.1. Starting Material

Conditioned medium containing 2% fetal bovine serum is obtained from an adherent human cell line expressing GA-EPO propagated in a Cell Cube (Costar). The medium is clarified by centrifugation followed by filtration through a 0.45 micron Nalgene filter unit.
Ultrafiltration/diafiltration is performed using a Pellicon tangential flow system (Millipore) fitted with a 30,000 molecular weight cutoff membrane. The equilibration and dialysis buffer is 20 mM MES, pH 6.2, containing 0.5% Tween 20.

### II.2. Heparin Chromatography

Heparin Chromatography is carried out using Pharmacia's GradiFrac System at 4oC. A 280 ml column (14 x 5 cm) of Heparin Sepharose® CL-6B (Pharmacia) is equilibrated with 20 mM MES, pH 6.2, containing 0.1% Tween 20 at a flow rate of 10 ml/min. Sample is applied to the column at 10 ml/min with a Pharmacia P50 pump. The column is then washed with equilibration buffer until the absorbance at 280 nm stabilizes. Loosely bound protein is eluted by washing with one column volume (280 ml) of 20 mM MES, pH 6.2, containing 0.1% Tween 20 and 0.05 M NaCl while collecting 12 ml fractions. The wash is followed by a two column volume (560 ml) linear gradient from 0.05 M to 0.35 M NaCl in 0.02 M MES, pH 6.2, containing 0.1% Tween 20, then washed in 0.35 M NaCl in 0.02 M MES, pH 6.2, containing 0.1% Tween 20 for one column volume (280 ml). The NaCl in the elution buffer is increased to 1.0 M NaCl over one-half column volume (140 ml), then held in this buffer for 1 column volume (280 ml).

Total protein is monitored by absorbance at 280 nm and salt concentration is followed by an in-line conductivity monitor (Figure 2). The flow-through and eluant fractions are analyzed for GA-EPO content by ELISA (R & D Systems), by SDS-PAGE, and Western Blot.

The Heparin column is regenerated by application of 560 ml of 20 mM Tris-Cl, pH 9.0, containing 4 M Urea and 1.4 M NaCl, followed by equilibration buffer until the conductivity and pH of the column effluent are equal to those of the equilibration buffer.

In one experiment, of the GA-EPO loaded onto the Heparin column, 81% was recovered in the elution pool. Use of this column resulted in removal of 86% of the contaminating protein (Table 1).

### EXAMPLE 3

### Use of Heparin Chromatography as a First Chromatographic Step in a Purification Process

### III.1. Starting Material

Conditioned serum free medium obtained from a human cell line expressing GA-EPO propagated in suspension in spinner flasks, is clarified by centrifugation followed by filtration through a 0.45 micron Nalgene filter unit. Ultrafiltration/diafiltration is performed using a Minitan tangential flow system (Millipore) fitted with a 10,000 molecular weight cutoff membrane. The equilibration and dialysis buffer is 20 mM MES, pH 6.2, containing 0.5% Tween 20.

### III.2. Heparin Chromatography

Heparin Chromatography is carried out using Pharmacia's FPLC System at 25oC. A 12 ml column (6 x 1.6 cm) of Heparin Sepharose® CL-6B (Pharmacia) is equilibrated with 20 mM MES, pH 6.2, containing 0.1% Tween 20 at 1 ml/min. Sample to be applied to the column is loaded at 1 ml/min by Superloop. The column is then washed with equilibration buffer until the absorbance at 280 nm stabilizes. Next, a five column volume (60 ml) linear gradient from 0 to 0.4 M NaCl in 0.02 M MES, pH 6.2, containing 0.1% Tween 20 is applied followed by a one column volume gradient to 1.0 M NaCl in 0.02 M MES, pH 6.2, containing 0.1% Tween 20, then held in this buffer for one column volume.

Total protein is monitored by absorbance at 280 nm. The flow-through and eluant fractions are analyzed for GA-EPO content by ELISA (R & D Systems)(Figure 3), and by SDS-PAGE, and Western Blot.

The Heparin Sepharose® column is regenerated by application of 60 ml 20 mM Tris-Cl, pH 9.0, containing 4 M Urea and 1.4 M NaCl, followed by a wash with equilibration buffer until the conductivity and pH of the column effluent are equal to those of the equilibration buffer.

In one experiment, of the GA-EPO loaded onto the Heparin column, 90% was recovered in the elution pool. Use of this column resulted in removal of 97% of the contaminating protein (Table 1).

### EXAMPLE 4

### Use of Heparin Chromatography as a First Chromatographic Step in a Purification Process

### IV.1. Starting Material

Conditioned medium containing 4% fetal bovine serum is obtained from an adherent human cell line expressing GA-EPO propagated in a Cell Cube (Costar). The media is clarified by centrifugation followed by filtration through a 0.45 micron Nalgene filter unit. Buffer exchange is accomplished by overnight dialysis (12,000 to 14,000 molecular weight cutoff membrane) against 20 mM Tris, pH 7.0, containing 0.1% Tween 20.

### IV.2. Heparin Chromatography

Heparin Chromatography is carried out using Pharmacia's FPLC System at 25oC. A 10 ml column (5 x 1.6 cm) of Heparin Sepharose® 6 Fast Flow (Pharmacia) is equilibrated with 20 mM Tris, pH 7.0, containing 0.1% Tween 20 at 4 ml/min. Sample is applied to the column at 4 ml/min. The column is then washed with equilibration buffer until the absorbance at 280 nm stabilizes. The wash is followed by a 10 column volume (100 ml) linear gradient from 0 to 0.35 M NaCl in 20 mM Tris, pH 7.0, containing 0.1% Tween 20. The elution buffer is then increased to 1.0 M NaCl in 20 mM Tris, pH 7.0, containing 0.1% Tween 20 over 5 ml then held in this buffer for two column volumes (20 ml).

Total protein is monitored by absorbance at 280 nm. The flow-through and eluant fractions are analyzed for GA-EPO content by ELISA (R & D Systems) (Figure 4), SDS-PAGE, and Western Blot.

In one experiment, of the GA-EPO loaded onto the Heparin column, 89% was recovered in the elution pool. Use of this column resulted in removal of 93% of the contaminating protein (Table 1).

The Heparin Sepharose® 6 Fast Flow column is regenerated by washing with 20 ml of 0.1 N NaOH followed by a wash with equilibration buffer until the conductivity and pH of the column effluent are equal to that of the equilibration buffer.

### EXAMPLE 5

### Use of Heparin Chromatography as an Intermediate Step in a Purification Process.

### V.1. Starting Material

Conditioned medium (400 ml) containing 2% fetal calf serum is obtained from an adherent human cell line expressing GA-EPO propagated in roller bottles and is clarified by centrifugation followed by filtration through a 0.45 micron Nalgene filter unit. The material is diluted 4-fold with water and loaded onto a 100 ml Pharmacia Streamline® DEAE column equilibrated in 20 mM Tris-Cl, pH 8.0. After washing with 2 liters of equilibration buffer; GA-EPO is step eluted with 20 mM Tris-Cl, pH 8.0, containing 1 M NaCl. The eluted material is pooled, adjusted to 3 M NaCl and applied to a 100 ml Pharmacia Phenyl Sepharose® 6 Fast Flow column equilibrated in phosphate buffered saline (Dulbecco) containing an additional 3 M NaCl. After washing with phosphate buffered saline containing an additional 0.5 M NaCl, GA-EPO is eluted with 10 mM NaOH, pH 12, containing 20% propylene glycol and 4 M urea. The elution pool is dialyzed against 20 mM Tris-Cl, pH 8.0, containing 0.05% Tween 20, then applied to a 20 ml Q-Sepharose® Fast Flow column equilibrated in the same buffer. GA-EPO is eluted from the column using a 10 column volume gradient from 0 to 1.0 M NaCl in the equilibration buffer. GA-EPO-containing fractions are pooled and dialyzed against 20 mM MES, pH 6.2, containing 0.05% Tween 20.

### V.2. Heparin Chromatography

Two 1 ml prepacked columns of Pharmacia HiTrap Heparin® are connected in tandem and equilibrated in 20 mM MES, pH 6.2, containing 0.05% Tween 20. Dialyzed material from the Q-Sepharose® Fast Flow column is loaded onto the tandem HiTrap Heparin® columns. The columns are then washed with equilibration buffer until the absorbance at 280 nm stabilizes. The wash is followed by a 30 column volume (60 ml) linear gradient from 0 to 0.6 M NaCl in 0.02 M MES, pH 6.2, containing 0.05% Tween 20.

Total protein is monitored by absorbance at 280 nm. The flow-through and eluant fractions are analyzed for GA-EPO content by ELISA (R & D Systems)(Figure 5), SDS-PAGE, and Western Blot.

In one experiment, of the GA-EPO loaded onto the HiTrap® columns, 88% was recovered in the elution pool. Use of this column resulted in removal of 78% of the contaminating protein (Table 1).

### EXAMPLE 6

### Use of Heparin Chromatography as an Intermediate Step in a Purification Process.

### VI.1. Starting Material

Conditioned medium (602 ml) containing 2% calf serum is obtained from an adherent human cell line expressing GA-EPO propagated in roller bottles and is clarified by centrifugation followed by filtration through a 0.45 micron Nalgene filter unit. The material is adjusted to 3 M NaCl then loaded onto a 100 ml Pharmacia Phenyl Sepharose® 6 Fast Flow column equilibrated in phosphate buffered saline (Dulbecco) containing an additional 3 M NaCl. After washing with phosphate buffered saline (Dulbecco) containing an additional 0.5 M NaCl followed by washing with 20 mM Tris, pH 8.0, GA-EPO is eluted with 10 mM NaOH, pH 12, containing 20% propylene glycol and 4 M urea. GA-EPO-containing fractions are pooled and dialyzed against 20 mM MES, pH 5.2 containing 0.05% Tween 20.

### VI.2. Heparin Chromatography

Two 1 ml prepacked columns of HiTrap Heparin® (Pharmacia) are connected in tandem and equilibrated in 20 mM MES, pH 5.2, containing 0.05% Tween 20. Dialyzed material from the Phenyl Sepharose® Fast Plow column is loaded onto the tandem HiTrap Heparin® columns. The columns are then washed with equilibration buffer until the absorbance at 280 nm stabilizes. The wash is followed by a 30 column volume (60 ml) linear gradient from 0 to 1 M NaCl in 0.02 M MES, pH 5.2, containing 0.05 % Tween 20.

Total protein is monitored by absorbance at 280 nm. The flow-through and eluant fractions are analyzed for GA-EPO content by ELISA (R & D Systems)(Figure 6), SDS-PAGE, and Western Blot.

In one experiment, of the GA-EPO loaded onto the HiTrap® columns, 79% was recovered in the elution pool. Use of this column resulted in removal of 82% of the contaminating protein (Table 1).

### EXAMPLE 7

### Purification of Erythropoietin Produced by Expression of the Human Erythropoietin Gene in CHO Cells: Use of Heparin Chromatography as a First Chromatographic Step in a Purification Process

### VII.1 Starting Material

Commercially available erythropoietin produced by expression of the human erythropoietin gene in CHO cells was added to diafiltered, unconditioned culture medium and subjected to purification by Heparin Chromatography as follows:

Unconditioned medium (10 ml) containing 2 % calf serum is buffer exchanged against 20 mM Tris-Cl, pH 7.0, containing 0.1% Tween 20 using a CentriPrep 30 unit (Amicon) until the conductivity is 2.2 mmho. The final retentate (about 10 ml) is diluted to 18 ml with water to achieve a conductivity of 1.6 mmho. One vial (500 U) of CHO cell-derived erythropoietin (R&D Systems, tissue culture grade) is dissolved in 100 µl of water and added to 0.9 ml of the buffer exchanged, diluted medium, then filtered through an Acrodisc 0.2 µm filter (Gelman).

### VII.2. Heparin Chromatography

A 1 ml HiTrap Heparin® (Pharmacia)column is equilibrated at room temperature in 20 mM Tris-Cl, pH 7.0, containing 0.1% Tween 20 (1 ml/min). The flow rate is 1 ml/min, 1 ml fractions are collected, and absorbance at 280 nm is monitored. The filtered sample (0.7 ml) is applied to the column, followed by a 10 column volume wash with the equilibration buffer. Erythropoietin is eluted with a 15 column volume linear gradient to 20 mM Tris-Cl, pH 7.0, containing 0.4 M NaCl and 0.1% Tween 20. The pool of erythropoietin is concentrated to 1.0 ml using a Centricon-10 unit (Amicon). Erythropoietin concentration is determined by ELISA (R & D systems) and total protein concentration of the erythropoietin pool is determined by BCA assay (Pierce).

In one experiment, essentially all of the erythropoietin applied to the Heparin HiTrap® column was recovered in the elution pool. Use of this column resulted in the removal of 91% of the contaminating protein (Table 1).

### EXAMPLE 8

### Further Purification of GA-EPO After Heparin Chromatography

The following procedures serve as examples of some of many possible purification steps which can be utilized after Heparin Chromatography. The following examples are not to be construed to limit the number of applicable steps but serve to illustrate the versatility of Heparin Chromatography as a step that is useful and compatible with a variety of purification procedures.

### VIII.1 Anion Exchange Chromatography

A 25 ml Q Sepharose® Fast Flow (Pharmacia) column is equilibrated with Q Sepharose equilibration buffer (20 mM Tris-Cl, pH 7.5, containing 0.1% Tween 20). The GA-EPO pool obtained after Heparin Chromatography is dialyzed against Q Sepharose equilibration buffer then loaded onto the Q Sepharose® column. The column is washed with the Q Sepharose equilibration buffer until the absorbance at 280 nm stabilizes. The bound protein is eluted with 20 mM Tris-Cl, pH 7.5, containing 0.4 M NaCl and 0.1% Tween 20.

The 0.4 M NaCl eluted protein peak is analysed for GA-EPO by ELISA (R & D Systems) and for total protein by absorbance at 280 nm. The flow through and wash fractions are similarly analyzed.

### VIII.2 Cation Exchange Chromatography

A 25 ml Mono S® column (Pharmacia) is equilibrated with Mono S equilibration buffer (20 mM Tris-Acetate, pH 4.5, containing 0.1% Tween 20). The GA-EPO pool obtained after Heparin Chromatography is dialyzed against Mono S equilibration buffer then loaded onto the Mono S® column. The column is washed with the Mono S equilibration buffer until the absorbance at 280 nm stabilizes. GA-EPO is eluted in a pH gradient from Mono S equilibration buffer at pH 4.5 to 20 mM Tris-Acetate, pH 8.0, containing 0.1% Tween, 20.

The eluted protein peak fractions are analyzed for GA-EPO by ELISA (R & D Systems) and total protein by absorbance at 280 nm. The flow through and wash fractions are similarly analyzed.

### VIII.3. Hydrophobic Interaction Chromatography

GA-EPO obtained after Heparin Chromatography is made 4 M in NaCl and brought to pH 5.5 by dropwise addition of 1 N HCl. The material is loaded onto a 50 ml Phenyl Sepharose® 6 Fast Flow low substitution column (Pharmacia) equilibrated in 20 mM MES, pH 5.5, containing 4 M NaCl. After washing with five column volumes of the equilibration buffer to remove unbound material, GA-EPO is eluted with 20 mM Tris-Acetate buffer, pH 8.0 containing 4 M urea and 20% propylene glycol.

The eluted protein peak fractions are analyzed for GA-EPO by ELISA (R & D Systems) and total protein by absorbance at 280 nm. The flow through and wash fractions are similarly analyzed.

### VIII.4. Preparative Gel Electrophoresis

GA-EPO obtained after Heparin Chromatography is concentrated to 2 ml, made 0.1 M in dithiothreitol and 1% in SDS, then heated to 100oC for 1 minute. The sample is loaded onto a preparative gel electrophoresis column (BioRad) prepared as described by the BioRad technical manual using a 12% acrylamide resolving gel. The eluted protein peak fractions are analyzed for GA-EPO by Western Blot and for total protein at absorbance 280 nm.

### VIII.5. Affinity Chromatography Using Immobilized Monoclonal Anti-Human EPO Antibodies.

The GA-EPO after Heparin Chromatography is dialyzed against equilibration buffer (20 mM Tris-Cl, pH 8.0, containing 0.2% Tween 20). The sample is applied to a 0.25 ml affinity column (3M Emphase resin, Pierce, washed in equilibration buffer) which contains 0.5 mg anti-human EPO antibodies (Genzyme) immobilized by manufacturer's specifications. After washing with the equilibration buffer, GA-EPO is eluted with 0.2 M acetic acid, pH 3.5 containing 0.15 M NaCl. Eluted material is collected in 200 microliter fractions in tubes containing 20 microliters of 1 M Tris, pH 8.0 and 2 microliters of Tween 20. The eluted protein peak is analyzed for GA-EPO by ELISA (R & D Systems) and for total protein by absorbance at 280 nm.

### EXAMPLE 9

### Use of Heparin Chromatography Resins from Various Manufacturers

Heparin Chromatography for erythropoietin purification can be performed utilizing heparin resins from a variety of manufacturers. Five different resins were selected for evaluating binding and loading capacity of GA-EPO derived from ultrafiltered/diafiltered cultured medium containing 2% fetal bovine serum: (1 and 2) Heparin Agarose 6XL® Type I and type III from American International Chemical (AIC); (3 and 4) Heparin Sepharose® Cl-6B and Heparin Sepharose® 6 Fast Flow from Pharmacia; and (5) Toyopearl AF-Heparin-650M® from TosoHaas.

Starting material and chromatographic conditions utilized were as described in Example 2 using from 1 ml to 7 ml columns. For all of the resins tested, recovery of GA-EPO was at least 70%. No significant difference was noticed in loading capacity among the different resins.

### Analysis Methods

### ELISA:

Quantification of erythropoietin can be conveniently measured by use of an Enzyme Linked Immunosorbent Assay (ELISA). One such commercially available ELISA kit for quantifying human erythropoietin is available as a kit (Quantikine IVD) from R&D Systems Inc. Another ELISA kit for quantifying erythropoietin is from Genzyme (Predicta Kit). Essentially, the ELISA assays are performed according to manufacturer specifications. Alternatively, suitable antibodies developed to recognized erythropoietin can be developed into similar ELISA systems for assay of amounts of erythropoietin present.

### Protein Assay:

Quantification of protein samples is well known in the art. Such procedures include the Lowry, Bradford, and BCA Protein Assays. Kits for determining the amount of protein in a sample are commercially available, such as BCA Protein Assay Reagent Method produced by Pierce Chemical Co. Quantification of protein can also be estimated by spectrophotometry at 280 nm as known by those skilled in the art.

### Specific Activity:

Specific activity for samples is calculated by the following formula: Specific Activity = Total Units of GA-EPO by ELISA/Total mg protein by BCA assay. A purified preparation of GA-EPO has a specific activity of about 200,000 ELISA units/mg protein by BCA assay. Specific activity using the ELISA to quantify units of GA-EPO is only indirectly linked to the actual biological activity of GA-EPO. A test generally performed to determine in vivo specific activity is the exhypoxic polycythemic mouse bioassay as is known by those skilled in the art (such an assay can determine the specific activity as IU/mg, as known by one skilled in the art).

### SDS PAGE and Western Blot:

Samples are prepared for SDS-PAGE and analyzed on 10% or 12% polyacrylamide gels as is known in the art. For example, gels are commercially available (e.g., BioRad), and modes of use are as described in the BioRad Mini-Protean® II Dual Slab Cell instructions (according to the method of Laemmli, U.K. Nature 227, 680 (1970)). Coomassie R-250, at about 0.1% concentration, can be used for total protein staining. Transfer of proteins to ImmobilonTM-P PVDF membrane (Millipore) can be performed with a BioRad Mini Trans-Blot® Electrophoretic Transfer Cell following the instruction manual using 12.5 mM Tris-Cl, pH 8.3, containing 96 mM glycine, 10% methanol, and 0.01% SDS. The proteins are transferred for 1 hour at 100 V in a BioRad Mini Trans-Blot® apparatus. The membrane can be blocked for 1 hour at room temperature with rotation, or overnight at 4oC with 5% (w/v) non-fat milk (Carnation) in TBS-Tween (20 mM Tris-Cl, pH 7.4, 0.15 M NaCl, 0.05% Tween 20). The membrane can then be washed briefly in TBS-Tween and incubated with rotation for one hour at room temperature in rabbit anti-human erythropoietin (R&D Systems) diluted 1/1000 with 2.5% (w/v) non-fat milk in TBS-Tween (final concentration 1 ug/ml). The membrane is washed extensively and developed by a method such as chemiluminescence using the Amersham ECLTM Kit and exposed on film. The lower limit, of detection of GA-EPO by this technique is about 10-20 ng (SDS-PAGE followed by western blot).

If the blot is to be reprobed with a different antibody the membrane can be stripped with 60 mM Tris-Cl, pH 7.5, containing 2.0% SDS for 2 hours at room temperature with rotation. The blot is reblocked with blocking buffer. The new primary antibody is then added as described above. For instance, detection of transferrin can be done using a sheep anti-human transferrin-HRP conjugate antibody (Biodesign). For detection of bovine albumin, a sheep anti-bovine albumin-HRP conjugate (Bethyl Labs) can be used. The blots are washed and developed as described above.

## Claims

1. A process for the purification of erythropoietins which comprises the steps of:
a) contacting a biological fluid containing erythropoietins with a heparin chromatographic support such that said erythropoietins bind to said heparin chromatographic support;
b) eluting said erythropoietins with an elution buffer; and
c) collecting said erythropoietins.

2. A process of claim 1 for inclusion in any point in a multistep process in the purification of erythropoietins.

3. A process of claim 2 which is the first chromatographic step in the purification of erythropoietins.

4. A process of any one of claims 1 to 3 for removing contaminating proteins of erythropoietins.

5. A process of any one of claims 2 to 4 wherein said multistep process includes one or more chromatographic steps selected from the group of ion exchange, hydrophobic interaction, size exclusion, reverse phase, or affinity chromatography.

6. A process of any one of claims 1 to 5 wherein said biological fluid is filtered, ultrafiltered, centrifuged or dialyzed before contacting said biological fluid with said heparin affinity support.

7. A process of any one of claims 1 to 5 which further comprises that said biological fluid is clarified before contacting said biological fluid with said heparin affinty support.

8. A process of any one of claims 1 to 7 which further comprises that binding of said erythropoietin molecules occurs in an equilibration buffer.

9. A process of any one of claims 1 to 8 in which binding of said erythropoietin molecules occurs at around a pH of 4.9 to 8.0.

10. A process of any one of claims 1 to 9 which further comprises eluting said erythropoietins by varying the elution buffer.

11. A process of any one of claims 8 to 10 wherein said buffer additionally contains an anti-adsorbent.

12. A process of claim 10 which further comprises that eluting said erythropoietins occurs by increasing the salt concentration of an alkali halide.

13. A process of any one of claims 1 to 12 wherein said biological fluid is a culture fluid.

14. A process of claim 13 wherein said culture fluid is clarified.

15. A process of any one of claims 1 to 14 wherein the biological fluid or the collected fluid containing erythropoietins is buffer exchanged.

16. A process of claim 15 wherein said buffer exchange is performed between a pH of 6 and 8.

17. A process of claim 15 wherein said buffer exchange is performed against a buffer of 0 to 200 mM salt.

18. A process of any one of claims 1 to 17 wherein said heparin affinity support is from a commercially available source.

## Patentansprüche

1. Verfahren zur Reinigung von Erythropoietinen, das die folgenden Schritte umfaßt:
a) Inkontaktbringen einer erythropoietinhaltigen biologischen Flüssigkeit mit einem Heparin-Chromatographieträger, so daß die Erythropoietine an den Heparin-Chromatographieträger binden,
b) Eluieren der Erythropoietine mit einem Elutionspuffer und
c) Gewinnen-der Erythropoietine.

2. Verfahren nach Anspruch 1 zum Einbau an einem beliebigen Punkt eines Mehrschrittverfahrens zur Reinigung von Erythropoietinen.

3. Verfahren nach Anspruch 2, das der erste Chromatographieschritt bei der Reinigung von Erythropoietinen ist.

4. Verfahren nach einem der Ansprüche 1 bis 3 zum Entfernen von Erythropoietine verunreinigenden Proteinen.

5. Verfahren nach einem der Ansprüche 2 bis 4, wobei das Mehrschrittverfahren einen oder mehrere chromatographieschritte einschließt, die ausgewählt -sind aus Ionenaustausch-, hydrophobe Wechselwirkungs-, Größenausschluß-, Umkehrphasen- oder Affinitätschromatographie.

6. Verfahren nach einem der Ansprüche 1 bis 5, wobei die biologische Flüssigkeit filtriert, ultrafiltriert, zentrifugiert oder dialysiert wird, bevor die biologische Flüssigkeit mit dem Heparinaffinitätsträger in Kontakt gebracht wird.

7. Verfahren nach einem der Ansprüche 1 bis 5, das weiter die Klärung der biologischen Flüssigkeit vor dem Inkontaktbringen der biologischen Flüssigkeit mit dem Heparinaffinitätsträger umfaßt.

8. Verfahren nach einem der Ansprüche 1 bis 7, das weiter die Bindung der Erythropoietinmoleküle in einem Äquilibrierungspuffer umfaßt.

9. Verfahren nach einem der Ansprüche 1 bis 8, wobei die Bindung der Erythropoietinmoleküle bei einem ungefähren pH-Wert von 4,9 bis 8,0 stattfindet.

10. Verfahren nach einem der Ansprüche 1 bis 9, das weiter die Elution der Erythropoietine durch Veränderung des Elutionspuffers umfaßt.

11. Verfahren nach einem der Ansprüche 8 bis 10, wobei der Puffer zusätzlich ein Antiadsorptionsmittel enthält.

12. Verfahren nach Anspruch 10, das weiter die Elution der Erythropoietine durch Erhöhung der Salzkonzentration eines Alkalihalogenids umfaßt.

13. Verfahren nach einem der Ansprüche 1 bis 12, wobei die biologische Flüssigkeit eine Kulturflüssigkeit ist.

14. Verfahren nach Anspruch 13, wobei die Kulturflüssigkeit geklärt wird.

15. Verfahren nach einem der Ansprüche 1 bis 14, wobei bei der erythropoietinhaltigen biologischen Flüssigkeit oder der erythropoietinhaltigen gewonnenen Flüssigkeit der Puffer ausgetauscht wird.

16. Verfahren nach Anspruch 15, wobei der Pufferaustausch bei einem pH-Wert von 6 bis 8 durchgeführt wird.

17. Verfahren nach Anspruch 15, wobei der Pufferaustausch durch einen Puffer mit 0 bis 200 mM Salz erfolgt.

18. Verfahren nach einem der Ansprüche 1 bis 17, wobei der Heparinaffinitätsträger im Handel erhältlich ist.

## Revendications

1. Procédé de purification d'érythropoïétines qui comprend les étapes suivantes :
a) la mise en contact d'un fluide biologique contenant des érythropoïétines avec un support chromatographique d'héparine de telle sorte que les érythropoïétines se lient audit support chromatographique d'héparine;
b) l'élution des érythropoïétines avec un tampon d'élution; et
c) la collecte desdites érythropoïétines.

2. Procédé suivant la revendication 1 pour l'inclusion en un point quelconque dans un procédé à étapes multiples dans la purification d'érythropoïétines.

3. Procédé suivant la revendication 2, qui est la première étape chromatographique dans la purification d'érythropoïétines.

4. Procédé suivant l'une quelconque des revendications 1 à 3, pour enlever les protéines de contamination des érythropoïétines.

5. Procédé suivant l'une quelconque des revendications 2 à 4, dans lequel le procédé à étapes multiples comprend une ou plusieurs étapes chromatographiques choisies dans le groupe comprenant les chromatographies d'échange ionique, d'interaction hydrophobe, d'exclusion en fonction de la taille, à phase inverse et d'affinité.

6. Procédé suivant l'une quelconque des revendications 1 à 5, dans lequel le fluide biologique est filtré, ultrafiltré, centrifugé ou dialysé avant la mise en contact dudit fluide biologique avec le support d'affinité d'héparine.

7. Procédé suivant l'une quelconque des revendications 1à 5, qui comprend de plus la clarification du fluide biologique avant la mise en contact dudit fluide biologique avec le support d'affinité d'héparine.

8. Procédé suivant l'une quelconque des revendications 1 à 7, qui comprend de plus la liaison des molécules d'érythropoïétine dans un tampon d'équilibre.

9. Procédé suivant l'une quelconque des revendications 1 à 8, dans lequel la liaison des molécules d'érythropoïétine se fait aux alentours d'un pH de 4,9 à 8,0.

10. Procédé suivant l'une quelconque des revendications 1 à 9, qui comprend de plus l'élution des érythropoïétines en faisant varier le tampon d'élution.

11. Procédé suivant l'une quelconque des revendications 8 à 10, dans lequel le tampon contient de plus un antiadsorbant.

12. Procédé suivant la revendication 10, dans lequel en outre l'élution des érythropoïétines se fait en augmentant la concentration en sel d'un halogénure d'alcali.

13. Procédé suivant l'une quelconque des revendications 1 à 12, dans lequel le fluide biologique est un fluide de culture.

14. Procédé suivant la revendication 13, dans lequel le fluide de culture est clarifié.

15. Procédé suivant l'une quelconque des revendications 1 à 14, dans lequel le fluide biologique contenant des érythropoïétines ou le fluide recueilli contenant des érythropoïétines est, échangé par tampon.

16. Procédé suivant la revendication 15, dans lequel le tampon échangé est réalisé entre un pH de 6 et 8.

17. Procédé suivant la revendication 15, dans lequel le tampon échangé est réalisé contre un tampon de sel de 0 à 200 mM.

18. Procédé suivant l'une quelconque des revendications 1 à 17, dans lequel le support d'affinité d'héparine est d'une origine disponible dans le commerce.
